# EUROPEAN PATENT SPECIFICATION

(11) **EP 1 184 413 B1**
(45) Date of publication and mention of the grant of the patent: **30.11.2005**
(21) Application number: 01307392.9
(22) Date of filing: 30.08.2001
(51) Int. Cl.: C08L 3/02, C12P 19/14, D21H 17/28, D21H 19/54

(54) **Enzymatic conversion of starch**
Enzymatische Umwandlung von Stärke
Conversion enzymatique de l'amidon

(30) Priority: 31.08.2000 GB 0021225
(43) Date of publication of application: 06.03.2002
(73) Proprietor: CERESTAR HOLDING B.V., NL-4551 LA Sas van Gent (NL)
(72) Inventor: Houghton, Robert William, Dukinfield, Cheshire SK16 5JG (GB); Embleton, Susan, Northfleet, Kent DA11 8LG (GB); Farrar, Mark, Heaton Mersey, Cheshire SK4 3DG (GB)
(74) Representative: Wilkinson, Stephen John

(56) References cited:
- EP-A- 0 049 009
- DATABASE CHEMABS [Online] CHEMICAL ABSTRACTS SERVICE, COLUMBUS, OHIO, US; HOHNEN OIL CO., LTD., JAPAN: "Corrugated paperboard adhesives for high-speed corrugators" retrieved from STN Database accession no. 96:70717 CA XP002160557 & JP 56 129278 A (HOHNEN OIL CO., LTD., JAPAN) 9 October 1981 (1981-10-09)

## Description

The present invention relates to the enzymatic conversion of starch. Starch is enzymatically hydrolysed to a product with a desired and stable end viscosity. The stable end viscosity is obtained by ending the enzymatic activity by heating the product in the presence of sodium perborate. More specifically the invention relates to a product comprising starch and sodium perborate. The addition of the perborate to the starch powder makes the subsequent enzymatic conversion of starch with alpha-amylase and the termination of the reaction much easier. The process results in a starch paste with a stable end viscosity. The product is preferably used in paper and corrugating industries, specifically in paper surface sizing and/or coating.

### Background of the invention

Starch is a carbohydrate synthesised in corn, tapioca, potato and most other plants out of dextrose units. The polymer exists in two forms: a linear structure of about 500 units, amylose and a branched structure of several thousand units, amylopectin. The relative amounts of amylose and amylopectin differ between different types of plants. The linear amylose constitutes 27% of normal corn starch, the branched amylopectin makes up the remaining 73%. Some corn species have been selected for their high amylose content they contain up to 70 % amylose.

Starch, starch derivatives and modified starches have found a large number of applications in such diverse fields as food, feed, detergents, pharmacy and the paper and corrugating industry.

In the field of corrugated paperboard adhesives, liquid mixtures of starch and sodium perborate are known.

JP 56129278 (Derwent Abstract) describes adhesives containing starch, sodium perborate, water and sodium hydroxide. These adhesives have low viscosity and high speed adhesion.

EP 0 049 009 describes a process for preparing starch glue from starch, in particular native starch whereof part of starch is gelatinised in water in an alkaline medium optionally followed by the addition of additives such as boron-chemicals. The dry material content of starch is 20-45% by weight and additives such as sodium perborate are added. Highly preferred is sodium perborate together with a copper sulfate catalyst.

The basic purpose for the use of starch in the paper coating industry is to bind the pigment particles to each other and to the paper substrate. In order to achieve this adhesive effect of starch, the starch has to be gelatinised. The usual method to gelatinise the starch is to heat the slurried starch above the temperature required for gelatinisation. Under these conditions the granules swell and fragments are formed resulting in a colloidal dispersion which then acts as an adhesive.

When native starch is simply cooked in this manner the resulting adhesive is much too viscous to meet the water balance requirements needed for high solids coating formulations. The so prepared starch is too viscous for applications and shows a very high set back behaviour.

The final viscosity of the starch can be influenced by chemically or thermally treating the starch prior to use. Depending on the properties desired, a number of chemical methods may be used by the starch supplier to modify the dry starch product. Alternatively, the user can employ a suitable conversion process (e.g. enzyme, thermal, thermal/chemical) when preparing the sizing solution. The starch cooking process may be either batch or continuous.

In general, for enzymatic conversion of native starch, alpha-amylase is used as the preferred hydrolytic enzyme. By varying the amount of enzyme, the reaction time or the temperature, different end viscosities can be obtained.

When the desired viscosity has been reached the reaction should be stopped. This can be done by increasing the temperature to a value at which the enzyme is inactivated. This is generally at temperatures higher than 95°C for the alpha-amylases employed in starch conversion. Since at somewhat elevated altitude this temperature cannot be reached in vessels at ambient pressure it may be desirable to use the other way of inactivating enzymes i.e. by addition of chemicals.

Chemicals, which may be used for this application are copper sulphate, sodium phosphate, and zinc containing compounds. In order not to influence the reaction these and similar compounds are added at the end of the enzymatic conversion that is in the slurry. The inactivation of the enzyme by the metal ions is generally performed with a concurrent increase in temperature.

The compounds containing heavy metals are not longer considered applicable for this purpose due to environmental constraints. Moreover, the paper manufacturers are faced with the problem of adding the chemicals in a controlled and dosed manner. This requires additional investment, manpower and maintenance.

A further alternative would be to change the pH in order to inactivate the enzymes. However, this would require a further addition of neutralising or buffering compounds as otherwise the quality of the starch paste would cause problems when used in the paper production process.

We have now found that a desired and stable starch end viscosity can be obtained which works under all ambient pressure conditions and does not require the addition of inactivation chemicals by the paper manufacturer. In addition the process of the present invention does not require the addition of chemicals which are not environmentally acceptable.

### Summary of the invention

The present invention discloses a dry composition comprising native starch and sodium perborate. The sodium perborate is present in an amount of from 0.1 to 1% w/w preferably from 0.1% to 0.5%. The composition is suited for the preparation of viscosity stable starch by enzymatic hydrolysis of the starch.

The present invention further discloses a process for preparing viscosity stable starch wherein use is made of a composition containing native starch and perborate. The process comprises the following steps:
a) preparing a slurry with a concentration from 25 to 38% solids, preferably 35% solids, containing native starch and from 0.1% w/w to 0.5% w/w sodium perborate based on native starch,
b) adding an effective amount of hydrolytic enzyme to the slurry,
c) heating the slurry to a temperature of between 65° and 80 °C,
d) keeping the slurry at the temperature of between 65° and 80 °C until a desired end viscosity is reached,
e) further increasing the temperature to a value which leads to enzyme inactivation, and optionally
f) allowing the starch slurry to cool.
   The current invention further relates to a process wherein the hydrolytic enzyme is alpha-amylase.
   The current invention relates to a process comprising the following steps:

a) preparing a slurry with a concentration from 25 to 38% solids, preferably 35% solids containing native starch and from 0.1% w/w to 0.5% w/w sodium perborate based on native starch,
b) adding from 0.01% to 0.1% of alpha-amylase to the slurry,
c) heating the slurry to a temperature of between 65° and 80 °C,
d) keeping the slurry at the temperature of between 65° and 80 °C until a desired end viscosity is reached,
e) further increasing the temperature to 85°C, preferably to 90°C, more preferably to 95°C which leads to alpha-amylase inactivation, and optionally
f) allowing the starch slurry to cool.

The current invention further relates to a process for inactivating hydrolytic enzymes, preferably alpha-amylase, in a starch slurry by sodium perborate characterised in that sodium perborate is added to native starch and the temperature is increased to a value of which leads to enzyme inactivation.

Furthermore, the current invention relates to a process for inactivating hydrolytic enzymes wherein the temperature is increased to 85°C, preferably to 90°C, more preferably to 95°C.

The viscosity stable starch obtained according to the new process may be used for the surface application and/or coating of paper.

### Brief description of drawing

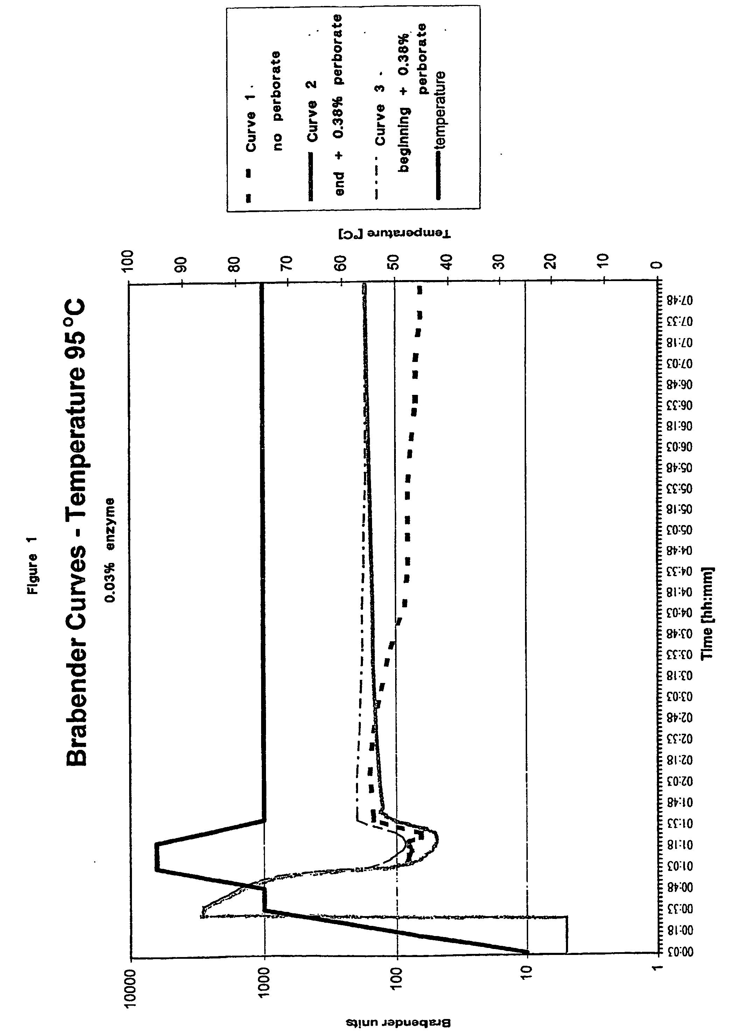

Figure 1 is a graph presenting the Brabender curves at a given temperature profile of 3 samples. Curve 1 is the Brabender profile of a starch slurry obtained after treatment with 0.03% enzyme without addition of sodium perborate. Curve 2 is the Brabender profile of a starch slurry obtained after treatment with 0.03% enzyme wherein 0.38% sodium perborate is added at the end of the experiment (conventional process). Curve 3 is a Brabender profile of a starch slurry obtained after treatment with 0.03% enzyme and 0.38% sodium perborate (added at the beginning - current disclosed process). Curve 1 demonstrates the instability of the viscosity of the starch thus obtained. Curve 2 and 3 show that the starches thus obtained have stable end viscosity.

### Detailed description of the invention

The present invention describes a process for obtaining a desired and stable starch end viscosity, which works under all ambient pressure conditions and does not require the addition of chemicals to halt the enzymatic activity, by the paper manufacturer. In addition the process of the present invention does not require the addition of chemicals which are not environmentally acceptable.

The present invention discloses a dry composition comprising native starch and sodium perborate. The native starch and sodium perborate mixture is dry, which makes shipping and storage easier. Furthermore such dry mixture is stable and in the dry composition, sodium perborate does not react with the native starch.

The dry mixture of starch/perborate is stored at the paper manufacturer until it is used. The paper manufacturer himself does not need an additional dosing facility in the deactivation process and he has no difficulties in dosing the effective amount of sodium perborate in a controlled and dosed manner. The sodium perborate is already present in the dry composition in such an amount that it is sufficient to inactivate the enzyme, which is used for the enzymatic conversion. The paper manufacturer just has to apply the dry composition of the current invention in the process for obtaining a desired and stable starch end viscosity.

The perborate is added into the enzyme conversion process for two reasons. Firstly, it inactivates via a free radical oxidation reaction the enzyme, thus halting the reduction in starch viscosity. Secondly, it raises the pH of the starch paste, preventing any risk of the paste causing thick coating mixes as a result of acid shock mechanisms when blended with pigment slurries.

The native starch is in principle any type of starch that is commercially available. Wheat, potato, tapioca, pea and corn starch can be used. The characteristics of the hydrolytic enzymes in use for the starch hydrolysis differ with respect to the required inactivation temperature. The enzymes used for corn starch conversion require a higher desactivation temperature and therefore the process of the present invention will be particularly useful when corn starch is used. The current process allows working at all ambient pressure conditions also for desactivation of hydrolytic enzymes requiring normally higher de-activation temperatures.

A suitable amount of the sodium perborate is from 0.1 to 1% w/w., preferably from 0.1% to 0.5%.

At the facilities of the paper manufacturer, the starch/perborate mixture (dry composition) is first dissolved to a certain dry solids content and the pH is adjusted to a value at which the enzymatic conversion is to take place. The optimum pH values for enzymatic conversion are given by the enzyme supplier and are generally between 6.5 and 7.5. A pH between 6.6 and 7.0 is mostly preferred.

The starch solids at which enzymatic conversion is made is determined by the total solids of the coating formula. Levels used for enzyme conversion for coating binder range from 25% to 38% starch, with a large part of the mills operating at around 35% solids. Lower starch solids are used for application as surface size.

To this starch slurry is added an effective amount of hydrolytic enzyme, preferably alpha-amylase. Alpha-amylases are available from different manufacturers under different trade names. Each manufacturer produces enzyme at his own level of enzymatic activity per unit weight, or in the case of liquid enzymes per unit of volume. Therefore the enzyme has to be tested before deciding on the amount required for use. In addition the solids concentration and temperature will also influence the performance and also have to be tested. Typical dosage levels of enzyme range from 0.01% to 0.1% on starch dry basis.

After addition of the enzyme the temperature of the slurry is increased and a specific time-temperature cycle is followed. The time-temperature process cycle is the basis of a successful enzymatic conversion resulting in a converted starch with a stable and pre-determined end viscosity. First the slurry is heated to reach the conversion temperature. The rate of heating has to be controlled to ensure that the heating is even without creating hot or cold areas, which would give rise to variations in the final product. Generally, the temperature is increased to a value between 65° and 80 °C. This temperature is maintained until the desired viscosity is achieved. The time to achieve this is between 15 min and 75 min. Preferably it is between 20 and 40 min. Sometimes it is preferred to perform the conversion at two different temperatures. After some time at a first conversion temperature the slurry is heated and the conversion is allowed to proceed at a higher temperature. When the final viscosity is reached the temperature is further increased to a value capable of inactivating the enzyme. This temperature is generally between 90 and 100°C. In fact, temperature is increased to at least 85°C, preferably temperature is increased to 90°C, more preferably to 95°C for inactivating the enzyme.

After cooling the starch may be stored although in general it is immediately used for surface sizing and/or paper coating applications.

The process for preparing viscosity stable starch comprises the following steps:
a) preparing a slurry with a concentration from 25 to 38% solids preferably 35% solids, containing native starch and from 0.1% w/w to 0.5% w/w sodium perborate based on native starch,
b) adding an effective amount of hydrolytic enzyme to the slurry,
c) heating the slurry to a temperature of between 65° and 80 °C,
d) keeping the slurry at the temperature of between 65° and 80 °C until a desired end viscosity is reached,
e) further increasing the temperature to a value of which leads to enzyme inactivation,
f) allowing the starch slurry to cool.

The current invention further relates to a process wherein in step b) the hydrolytic enzyme is alpha-amylase.
The current invention relates to a process comprising the following steps:
a) preparing a slurry with a concentration from 25 to 38% solids preferably 35% solids, containing native starch and from 0.1% w/w to 0.5% w/w sodium perborate based on native starch,
b) adding from 0.01% to 0.1% of alpha-amylase to the slurry,
c) heating the slurry to a temperature of between 65° and 80 °C,
d) keeping the slurry at the temperature of between 65° and 80 °C until a desired end viscosity is reached,
e) further increasing the temperature to 85°C, preferably to 90°C, more preferably to 95°C which leads to alpha-amylase inactivation, and optionally
f) allowing the starch slurry to cool.

Surprisingly the use of sodium perborate under the present conditions does not negatively influence the conversion reaction. During the whole enzymatic process, the sodium perborate does not influence the enzymatic conversion and by increasing the temperature at the end of the conversion, the perborate starts deactivating the enzyme.

The current invention further relates to a process for inactivating hydrolytic enzymes, preferably alpha-amylase, in a starch slurry by sodium perborate characterised in that sodium perborate is added to native starch and the temperature is increased to a value of which leads to enzyme inactivation. The inactivation of hydrolytic enzymes such as alpha-amylase can be obtained by adding prior to the enzymatic conversion sodium perborate to the slurry of native starch. After the enzymatic conversion the temperature is increased and the sodium perborate starts deactivating the enzyme.

Furthermore, the current invention relates to a process for inactivating hydrolytic enzymes wherein the temperature is increased to 85°C, preferably to 90°C, more preferably to 95°C.

The viscosity stable starch obtained according to the new process may be used for the surface application and/or coating of paper.

The current invention has the following advantages:
- the dry composition of native starch and sodium perborate is stable
- the presence of sodium perborate does not influence negatively the enzymatic conversion
- the paper manufacturer does not need a separate dosing facility and does not have to bother with the controlled dosing of sodium perborate
- the dry composition can be applied for inactivation of hydrolytic enzymes
- a slight temperature increase is sufficient for starting the deactivation of the enzyme by the sodium perborate already present in the mixture.

The present invention is illustrated by the following examples. From the examples it can be seen that the final viscosity is stable and that it can be easily regulated. The product and process of the present invention make handling of the starch conversion at the paper mills much simpler. In addition sodium perborate does not cause any environmental problems. Moreover, it makes it possible to perform the atmospheric conversion at high altitude and with corn starch. The enzyme for the conversion of corn starch is known to require a higher inactivation temperature than those enzymes used for converting potato or wheat starch.

The experiment demonstrates the need to add perborate to ensure that the enzyme is deactivated, thus preventing further starch hydrolysis. From the results of the overnight storage of the first sample (Sample 1) it can be seen that the starch viscosity is reducing due to storage, when compared with the second and third samples.

The experiment also surprisingly shows that adding the perborate into the starch slurry appears to have little effect on the enzyme activity on the starch. This is demonstrated when comparing results of Samples 2 and 3. This makes it possible to add the perborate to the starch when both are in a dry form. The paper or board manufacturer therefore does not have to add the chemical himself after the heating stage.

The experiment shows that by adding perborate into the starch slurry, the resulting starch pastes are buffered, and are stable, showing relatively little set-back tendencies.

Example 2 demonstrates (see Brabender curves of Figure 1) that at 95°C the current disclosed composition allows obtaining a stable end viscosity. The product obtained by applying the currently disclosed process has a stable end viscosity (curve 3). The result is similar to the enzyme inactivation by adding at the end sodium perborate (curve 2). The incativation of the enzyme is incomplete when no sodium perborate is added (curve 1).

### Experimental procedure

A standard laboratory enzyme procedure was used for all this work. In each test, 600 ml of starch slurry was prepared at 28% solids. The enzyme used was Aquazym 60 (supplied by TS Chemicals) and the starch was a food grade native maize product (type 03402, supplied by Cerestar). The enzyme was diluted 1:100 to make the addition levels more convenient.
The experiments were carried out in a waterbath, with the samples being stirred to ensure thorough mixing. Initial heating of samples was up to 75°C, holding at this temperature for 20 minutes. Finally, the paste temperature was increased to 92°C, and this temperature was held for a further 20 minutes.

The starch paste viscosity was measured on samples of the material so obtained as Brookfield RV at 100 rpm.

### Examples

### Example 1

Three different samples were prepared in the exercise. All three samples use the same starch slurry solids (28%) and the same enzyme dose (0.01% w/w, based on commercial starch weight). The difference between the samples concerns the perborate addition. In the first case, no perborate is added. In the second, perborate is added into the starch paste after the first heating stage (as in the conventional process). Finally, in the third, the perborate is added into the starch slurry before the first heating. In the second and third cases, exactly the same amount of perborate was used (0.25% w/w, based on commercial starch basis)

### Sample 1 - No perborate added

As made -
- Starch paste viscosity - 57 mPas at 80°C
- Starch paste pH - 6.1

After 2 hours storage, stirred, at 80°C -
- Starch paste viscosity - 68 mPas at 70°C

After overnight storage, stirred, at 80°C -
- Starch paste viscosity - 91 mPas at 70°C
   105 mPas at 60°C
   124 mPas at 50°C
   147 mPas at 40°C
   186 mPas at 30°C
- Starch paste dry solids - 29.8%
- Starch paste pH - 5.9
- Starch paste RAL colour - 1015

### Sample 2 - Perborate added to paste after first heating stage

As made -
- Starch paste viscosity - 55 mPas at 80°C
- Starch paste pH - 7.1

After 2 hours storage, stirred, at 80°C -
- Starch paste viscosity - 80 mPas at 70°C

After overnight storage, stirred, at 80°C -
- Starch paste viscosity - 118 mPas at 70°C
   135 mPas at 60°C
   170 mPas at 50°C
   248 mPas at 40°C
   366 mPas at 30°C
- Starch paste dry solids - 31.3%
- Starch paste pH - 6.3
- Starch paste RAL colour - 1014

### Sample 3 - Perborate added to the starch slurry before heating (new process)

As made -
- Starch paste viscosity - 72 mPas at 80°C
- Starch paste pH - 7.0

After 2 hours storage, stirred, at 80°C -
- Starch paste viscosity - 84 mPas at 70°C

After overnight storage, stirred, at 80°C -
- Starch paste viscosity - 116 mPas at 66°C
   133 mPas at 60°C
   165 mPas at 50°C
   233 mPas at 40°C
   378 mPas at 30°C
- Starch paste dry solids - 30.8%
- Starch paste pH - 6.3
- Starch paste RAL colour - 1014

This experiment demonstrates the need to add perborate to ensure that the enzyme is deactivated, thus preventing further starch hydrolysis. From the results of the overnight storage of the first sample (Sample 1) it can be seen that the starch viscosity is reducing due to storage, when compared with the second and third samples.

The experiment also surprisingly shows that adding the perborate into the starch slurry appears to have little effect on the enzyme activity on the starch. Since the deactivation of the enzyme is relying on the generation of free radicals from the perborate, it would appear that few, if any, of these free radicals are present when the starch paste is held at the first reaction temperature of 75°C.

The experiment shows that by adding perborate into the starch slurry, the resulting starch pastes are buffered, and are stable, showing relatively little set-back tendencies.

The experiment was repeated, but the enzyme addition was reduced to 0.01% w/w on commercial starch weight. The results were comparable with the results shown above.

### Example 2

The experimental procedure was identical to the experimental procedure for example 1, except that the paste temperature was increased to 95°C.
Three different samples were prepared. All three samples use the same starch slurry solids (28%) and the same enzyme dose (0.03% w/w, based on commercial starch weight). The difference between the samples concerns the perborate addition. In the first case, no perborate is added. In the second, perborate is added into the starch paste after the first heating stage (as in the conventional process). Finally, in the third, the perborate is added into the starch slurry before the first heating. In the second and third cases, exactly the same amount of perborate was used (0.38% w/w, based on commercial starch basis).

The samples were characterised by Brabender curves - see Figure 1.
Curve 1 demonstrates the instability of the viscosity of the starch thus obtained. Curve 2 and 3 show that the starches thus obtained have stable end viscosity.

## Claims

1. A dry composition comprising native starch and sodium perborate.

2. A dry composition according to claim 1 wherein the sodium perborate is present in an amount of from 0.1 to 1% w/w preferably from 0.1% to 0.5%.

3. A dry composition according to claim 1 wherein the starch is selected from the group consisting of wheat, potato, tapioca, pea and corn starch, preferably corn starch.

4. A process for preparing viscosity stable starch comprising the following steps:
a) preparing a slurry with a concentration 25 to 38% solids preferably 35% solids, containing native starch and from 0.1% w/w to 0.5% w/w sodium perborate based on native starch,
b) adding an effective amount of hydrolytic enzyme to the slurry,
c) heating the slurry to a temperature of between 65° and 80 °C,
d) keeping the slurry at the temperature of between 65° and 80 °C until a desired end viscosity is reached,
e) further increasing the temperature to a value of which leads to enzyme inactivation, and optionally
f) allowing the starch slurry to cool.

5. A process according to claim 4 **characterised in that** in step b) the hydrolytic enzyme is alpha-amylase.

6. A process according to claim 4 or 5 **characterised in that** it is comprising the following steps:
a) preparing a slurry with a concentration 25 to 38% solids, preferably 35% solids, containing native starch and from 0.1 % w/w to 0.5% w/w sodium perborate based on native starch,
b) adding from 0.01% to 0.1% of alpha-amylase to the slurry,
c) heating the slurry to a temperature of between 65° and 80°C,
d) keeping the slurry at the temperature of between 65° and 80 °C until a desired end viscosity is reached,
e) further increasing the temperature to 85°C, preferably to 90°C, more preferably to 95°C which leads to alpha-amylase inactivation, and optionally
f) allowing the starch slurry to cool.

7. Process for inactivating hydrolytic enzymes, preferably alpha-amylase, in a starch slurry by sodium perborate **characterised in that** sodium perborate is added to native starch and the temperature is increased to a value which leads to enzyme inactivation.

8. A process according to claim 7 **characterised in that** the temperature is increased to 85°C, preferably to 90°C, more preferably to 95°C.

## Patentansprüche

1. Trockenzusammensetzung, umfassend natürliche Stärke und Natriumperborat.

2. Trockenzusammensetzung nach Anspruch 1, wobei Natriumperborat in einer Menge von 0,1 bis 1 Gew.-%, bevorzugt 0,1 bis 0,5 Gew.-% vorliegt.

3. Trockenzusammensetzung nach Anspruch 1, wobei die Stärke aus der Gruppe, bestehend aus Weizen-, Kartoffel-, Tapioka-, Erbsen- und Maisstärke, bevorzugt Maisstärke, ausgewählt ist.

4. Verfahren zur Herstellung viskositätsstabiler Stärke, umfassend die folgenden Schritte:
a) Herstellen einer Aufschlämmung mit einer Konzentration von 25 bis 38 % Feststoffen, bevorzugt 35 % Feststoffen, enthaltend natürliche Stärke und 0,1 bis 0,5 Gew.-% Natriumperborat, basierend auf natürlicher Stärke,
b) Zugeben einer wirksamen Menge an hydrolytischem Enzym zu der Aufschlämmung,
c) Erwärmen der Aufschlämmung auf eine Temperatur zwischen 65 und 80 °C,
d) Halten der Aufschlämmung bei der Temperatur zwischen 65 und 80 °C bis eine gewünschte Endviskosität erreicht ist,
e) weitere Erhöhung der Temperatur auf einen Wert, der zur Enzyminaktivierung führt, und gegebenenfalls
f) Abkühlenlassen der Stärkeaufschlämmung.

5. Verfahren nach Anspruch 4, **dadurch gekennzeichnet, daß** in Schritt b) das hydrolytische Enzyme alpha-Amylase ist.

6. Verfahren nach Anspruch 4 oder 5, **dadurch gekennzeichnet, daß** es die folgenden Schritte umfaßt:
a) Herstellen einer Aufschlämmung mit einer Konzentration von 25 bis 38 % Feststoffen, bevorzugt 35 % Feststoffen, enthaltend natürliche Stärke und 0,1 bis 0,5 Gew.-% Natriumperborat, basierend auf natürlicher Stärke,
b) Zugeben von 0,01 bis 0,1 % alpha-Amylase zu der Aufschlämmung,
c) Erwärmen der Aufschlämmung auf eine Temperatur zwischen 65 und 80 °C,
d) Halten der Aufschlämmung bei der Temperatur zwischen 65 und 80 °C bis eine gewünschte Endviskosität erreicht ist,
e) weitere Erhöhung der Temperatur auf 85 °C, bevorzugt 90 °C, stärker bevorzugt 95 °C, was zur alpha-Amylaseinaktivierung führt, und gegebenenfalls
f) Abkühlenlassen der Stärkeaufschlämmung.

7. Verfahren zur Inaktivierung hydrolytischer Enzyme, bevorzugt alpha-Amylase, in einer Stärkeaufschlämmung durch Natriumperborat, **dadurch gekennzeichnet, daß** Natriumperborat zu natürlicher Stärke zugegeben wird, und die Temperatur auf einen Wert erhöht wird, der zur Enzyminaktivierung führt.

8. Verfahren nach Anspruch 7, **dadurch gekennzeichnet, daß** die Temperatur auf 85 °C, bevorzugt 90 °C, stärker bevorzugt 95 °C erhöht wird.

## Revendications

1. Composition sèche comprenant de l'amidon.natif et du perborate de sodium.

2. Composition sèche selon la revendication 1 dans laquelle le perborate de sodium est présent en une quantité allant de 0,1 à 1% en poids et de préférence allant de 0,1% à 0,5%.

3. Composition sèche selon la revendication 1 dans laquelle l'amidon est choisi dans le groupe constitué par le blé, les pommes de terre, le tapioca, l'amidon de pois et de maïs, de préférence de l'amidon de maïs.

4. Procédé de préparation d'un amidon de viscosité stable comprenant les étapes suivantes :
a) préparer une bouillie avec une concentration de 25 à 38% de solides de préférence de 35% de solides, contenant de l'amidon natif et allant de 0,1% en poids à 0,5% en poids de perborate de sodium en se basant sur l'amidon natif,
b) ajouter une quantité efficace d'enzyme hydrolytique à la bouillie,
c) chauffer la bouillie à une température comprise entre 65° et 80°C,
d) garder la bouillie à la température comprise entre 65° et 80°C jusqu'à ce qu'une viscosité finale désirée soit atteinte,
e) monter encore en température à une valeur qui mène à l'inactivation de l'enzyme, et éventuellement
f) laisser la bouillie d'amidon refroidir.

5. Procédé selon la revendication 4 **caractérisé en ce que** dans l'étape b) l'enzyme hydrolytique est l'alpha-amylase.

6. Procédé selon la revendication 4 ou 5 **caractérisé en ce qu'**il comprend les étapes suivantes :
a) préparer une bouillie avec une concentration de 25 à 38% de solides, de préférence de 35% de solides, contenant de l'amidon natif et de 0,1% en poids à 0,5% en poids de perborate de sodium en se basant sur l'amidon natif,
b) ajouter de 0,01% à 0,1% d'alpha-amylase à la bouillie,
c) chauffer la bouillie à une température comprise entre 65° et 80°C,
d) garder la bouillie à la température comprise entre 65° et 80°C jusqu'à ce qu'une viscosité finale désirée soit atteinte,
e) augmenter encore la température jusqu'à 85°C, de préférence à 90°C, encore plus préférable à 95°C ce qui mène à l'inactivation de l'alpha-amylase, et éventuellement
f) laisser la bouillie d'amidon refroidir.

7. Procédé pour inactiver les enzymes hydrolytiques, de préférence l'alph-amylase, dans une bouillie d'amidon avec du perborate de sodium **caractérisé en ce que** le perborate de sodium est ajouté à l'amidon natif et la température est augmentée jusqu'à une valeur menant à l'inactivation de l'enzyme.

8. Procédé selon la revendication 7 **caractérisé en ce que** la température est augmentée jusqu'à 85°C, de préférence à 90°C, encore plus préférable à 95°C.
